(19) Europäisches Patentamt
European Patent Office
Office européen des brevets

(11) Veröffentlichungsnummer: **0 110 259**
**B1**

(12) # EUROPÄISCHE PATENTSCHRIFT

(45) Veröffentlichungstag der Patentschrift: **17.12.86**

(51) Int. Cl.⁴: **C 07 D 211/90**, A 61 K 31/445

(21) Anmeldenummer: **83111521.7**

(22) Anmeldetag: **18.11.83**

(54) **1,4-Dihydropyridinderivate, Verfahren zu ihrer Herstellung sowie ihre Verwendung in Arzneimitteln.**

(30) Priorität: **30.11.82 DE 3244178**

(43) Veröffentlichungstag der Anmeldung: **13.06.84 Patentblatt 84/24**

(45) Bekanntmachung des Hinweises auf die Patenterteilung: **17.12.86 Patentblatt 86/51**

(84) Benannte Vertragsstaaten: **AT BE CH DE FR GB IT LI NL SE**

(56) Entgegenhaltungen:
**EP-A-0 012 180**
**EP-A-0 092 936**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Wehinger, Egbert, Dr., Gellertweg 33, D-5600 Wuppertal 1 (DE)**
Erfinder: **Meyer, Horst, Dr., Henselweg 11, D-5600 Wuppertal 1 (DE)**
Erfinder: **Kazda, Stanislav, Dr., Gellertweg 18, D-5600 Wuppertal 1 (DE)**
Erfinder: **Knorr, Andreas, Dr., Pahlkestrasse 15, D-5600 Wuppertal 1 (DE)**

EP 0 110 259 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft 1,4-Dihydropyridin-derivate, mehrere Verfahren zu ihrer Herstellung sowie ihre Verwendung in Arzneimitteln, insbesondere in kreislaufbeeinflussenden Mitteln.

Es ist bereits bekannt geworden, daß man 1,4-Dihydro-2,6-dimethyl-4-phenyl-pyridin-3,5-dicarbonsäurediethylester erhält, wenn man 2-Benzylidenacetessigsäureethyl-ester mit B-Aminocrotonsäureethylester oder Acetessigsäureethylester und Ammoniak umsetzt [E. Knoevenagel, Der. dtsch- chem. Ges. 31, 743 (1898)].

Weiterhin ist bekannt, daß bestimmte 1,4-Dihydropyridine interessante pharnakologische Eigenschaften aufweisen [F. Bossert, w. Vater, Naturwissenschaften 58, 578 (1971)].

Ebenso ist es aus der deutschen Offenlegungsschrift 28 47 236 bekannt, daß ähnliche Verbindungen als Coronarmittel und als blutdrucksenkende Mittel verwendet werden können.

In der älteren Europäischen Patentanmeldung Nr. 83302075.3 der Taisho Pharmaceutical Co. Ltd. werden 1,4-Dihydro-2,6-dimethylpyridin-3,5-dicarbonsäureester beschrieben, die in 4-Position einen Nitrophenylrest tragen und deren Estergruppen in 3- und 5-Position maximal 4 C-Atome enthalten. Diese Anmeldung gilt als Stand der Technik gemäß Artikel 54 Abs. 3 EPÜ.

Die Erfindung betrifft I,4-Dihydropyridine der allgemeinen Formel I

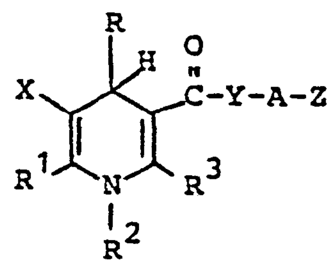

(I)

in welcher

R für Pyridyl, Benzoxadiazolyl oder Phenyl steht, welches I- bis 2-fach substituiert ist durch Nitro, Trifluormethyl, Halogen, Cyano und/oder Azido,

$R^1$ und $R^3$ gleich oder verschieden sind und jeweils $C_1$-$C_4$-Alkyl oder Benzyl bedeuten,

$R^2$ Wasserstoff, $C_1$-$C_4$-Alkyl oder Benzyl darstellt,

Y Sauerstoff ist,

A $C_1$-$C_4$-Alkylen bedeutet,

Z $-ONO_2$ darstellt,

X für $-COR^4$ mit $R^4$ gleich $C_1$-$C_4$-Alkyl, oder für $-COOR^5$ steht, wobei $R^5$ Benzyl oder geradkettiges, verzweigtes oder cyclisches Alkyl mit bis zu 12 C-Atomen bedeutet, das gegebenenfalls durch ein Sauerstoffatom in der Alkylkette unterbrochen ist oder gegebenenfalls substituiert ist durch Halogen und/oder durch Amino, wobei die Aminogruppe zwei gleiche oder verschiedene Substituenten aus der Gruppe $C_1$-$C_4$-Alkyl und Benzyl aufweist, wobei für den Fall, daß R für Nitrophenyl steht $R^5$ mindestens 5 C-Atome enthält, oder für die Gruppe -CO-Y'-A'-Z' steht, wobei diese Gruppe mit -CO-Y-A-Z gleich oder verschieden sein kann und wobei die Definition von Y', A' und Z' der von Y, A und Z entspricht,

sowie deren pharmazeutisch unbedenkliche Säureadditionssalze.

Bevorzugt seien Verbindungen der allgemeinen Formel I genannt, in welcher

R Nitrophenyl, der Trifluormethylphenyl bedeutet,

$R^1$ und $R^3$ jeweils Methyl darstellen,

$R^2$ Wasserstoff ist,

Y Sauerstoff bedeutet,

A $C_1$-$C_3$-Alkyle, darstellt,

Z $-ONO_2$ ist,

X $-COR^4$ mit $R^4$ gleich Methyl bedeutet oder für $-COOR^5$ steht wobei $R^5$ geradkettiges verzweigtes oder cyclisches Alkyl mit bis zu 12 C-Atomen, gegebenenfalls durch ein Sauerstoffatom in der Alkylkette unterbrochen, oder Benzyl darstellt, wobei für den Fall, daß R für Nitrophenyl steht, $R^5$ mindestens 5 C-Atome enthält.

Es wurde weiterhin gefunden, daß man die 1,4-Dihydro-pyridinderivate der allgemeinen Formel I erhält, wenn man

A) Ylidenverbindungen der Formel II,

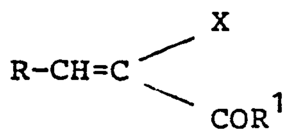

(II)

in welcher
R, $R^1$ und X die oben angegebene Bedeutung haben,
mit Enaminocarbonsäurederivaten der Formel III

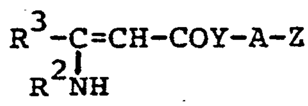

(III)

in welcher
$R^2$, $R^3$, Y, A und Z die oben angegebene Bedeutung haben,
gegebenenfalls in Gegenwart inerter organischer Lösungsmittel umsetzt, oder
B) Ylidenverbindungen der Formel II

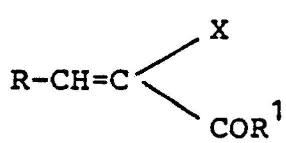

(II)

in welcher
R, $R^1$ und X die oben angegebene Bedeutung haben,
mit Aminen der Formel IV und β-Ketocarbonsäurederivaten der Formel V

$$R^2\text{-}NH_2 \qquad\qquad R^3\text{-}CO\text{-}CH_2\text{-}COY\text{-}A\text{-}Z$$

(IV) (V)

in welchen
$R^2$, $R^3$, Y, A und Z die oben angegebene Bedeutung haben, gegebenenfalls in Gegenwart inerter organischer Lösungsmittel umsetzt, oder
C) Yliden-β-dicarbonylverbindungen der Formel VI

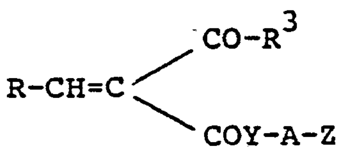

(VI)

in welcher
R, $R^3$, Y, A und Z die oben angegebene Bedeutung haben, mit Enaminoverbindungen der Formel VII

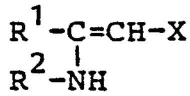

(VII)

in welcher
$R^1$, $R^2$ und X die oben angegebene Bedeutung haben, gegebenenfalls in Gegenwart inerter organischer Lösungsmittel zur Reaktion bringt, oder
D) Yliden-β-dicarbonylverbindungen der Formel VI

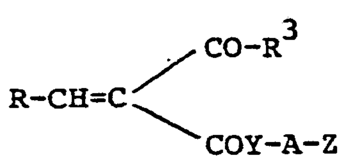

(VI)

in welcher
R, $R^3$, Y, A und Z die oben angegebene Bedeutung haben,

mit Aminen der Formel IV und Keto-Derivaten der Formel VIII

$R^2-NH_2$ (IV)

$R^1-CO-CH_2-X$ (VIII)

in welchen
$R^2$, $R^1$ und X die oben angegebene Bedeutung haben,
gegebenenfalls in Gegenwart inerter organischer Lösungsmittel umsetzt, oder
E) Aldehyde der Formel IX

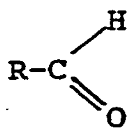 (IX)

in welcher
R die oben angegebene Bedeutung hat,
mit Enaminoverbindungen der Formel VII

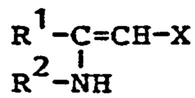 (VII)

in welcher
$R^1$, $R^2$ und X die oben angegebene Bedeutung haben,
und β-Ketocarbonsäurederivaten der Formel V

$R^3-CO-CH_2-COY-A-Z$ (V)

in welcher
$R^3$, Y, A und Z die oben angegebene Bedeutung haben,
gegebenenfalls in Gegenwart inerter organischer Lösungsmittel umsetzt, oder
F) Aldehyde der Formel IX

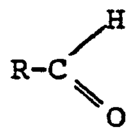

(IX)

in welcher
R die oben angegebene Bedeutung hat,
mit Enaminocarbonsäurederivaten der Formel III

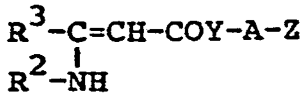

(III)

in welcher
$R^2$, $R^3$, Y, A und Z die oben angegebene Bedeutung haben,
und Keto-Derivaten der Formel VIII

$$R^1\text{-CO-CH}_2\text{-X} \qquad \text{(VIII)}$$

in welcher
$R^1$ und X die oben angegebene Bedeutung haben,
gegebenenfalls in Gegenwart inerter organischer Lösungsmittel umsetzt, oder wenn man
G) in 1,4-Dihydropyridinderivaten der Formel X

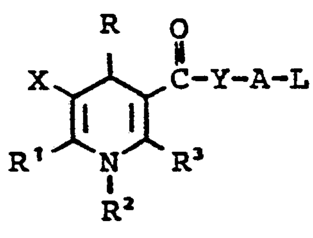

(X)

in welcher
R, $R^1$, $R^2$, $R^3$, A, X und Y die oben angegebene Bedeutung haben,
und
L eine geeignete austretende Gruppe, die beispielsweise Halogen darstellt,
diese Gruppe L gegen den Rest $-O-NO_2$ austauscht.

Die Salze der Verbindungen der allgemeinen Formel I können in einfacher Weise nach üblichen Salzbildungsmethoden z.B. durch Lösen der Base und Hinzufügen der Säure z.B. Chlorwasserstoff, erhalten werden und in bekannter Weise, z.B. durch Abfiltrieren, isoliert und gegebenenfalls gereinigt werden.

Die erfindungsgemäßen 1,4-Dihydropyridin-Derivate besitzen wertvolle pharmakologische Eigenschaften.

Aufgrund ihrer kreislaufbeeinflussenden Wirkung können sie als antihypertensive Mittel, als periphere und cerebrale Vasodilatatoren sowie als Coronartherapeutika Verwendung finden und sind somit als Bereicherung der Pharmazie anzusehen.

Je nach Art der verwendeten Ausgangsstoffe kann die Synthese der erfindungsgemäßen Verbindungen durch folgende Formelschemata wiedergegeben werden, wobei der 1,4-Dihydro-2,6-dimethyl-4-(3-nitrophenyl)-pyridin-3,5-dicarbonsäure-pentyl-(2-nitrooxyethyl)-ester als Beispiel gewählt sei:

A)

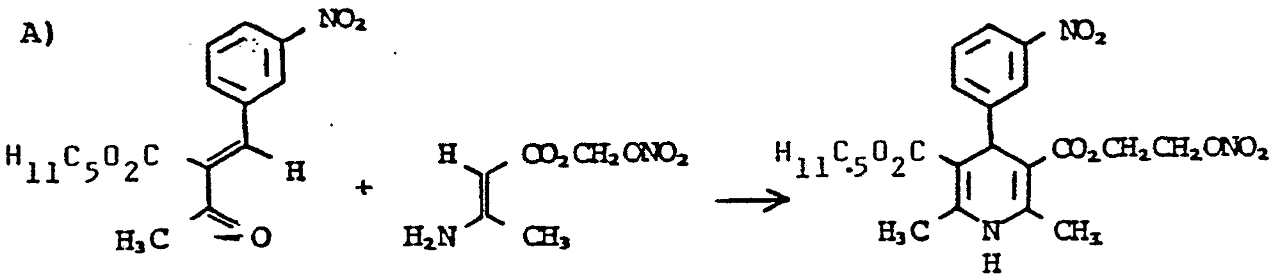

B)

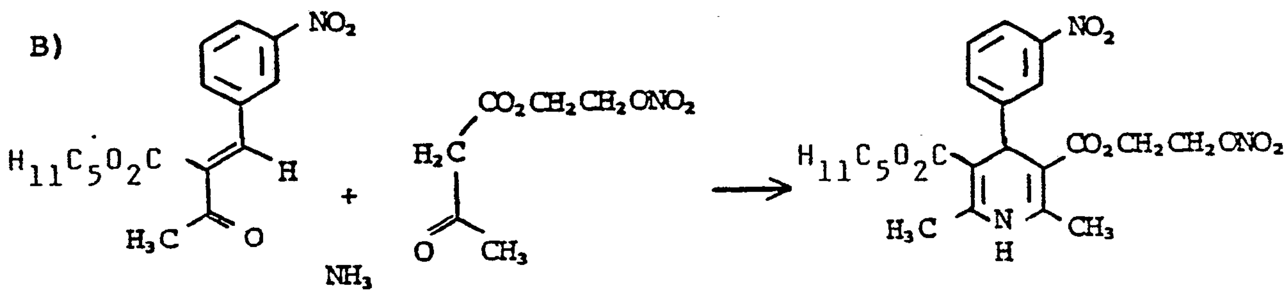

C)

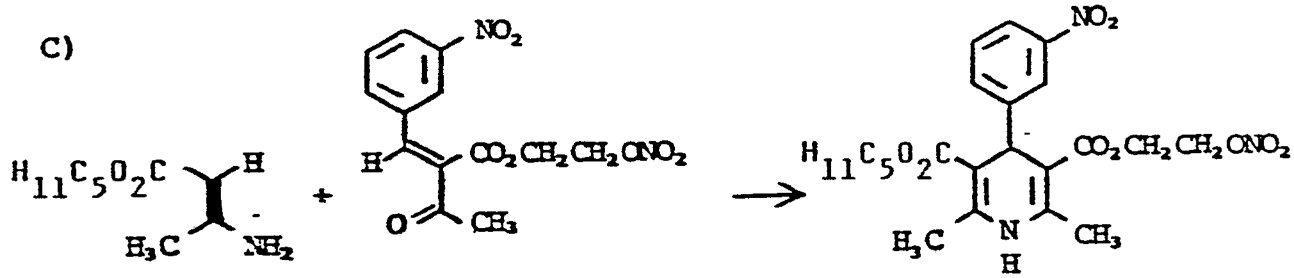

D)

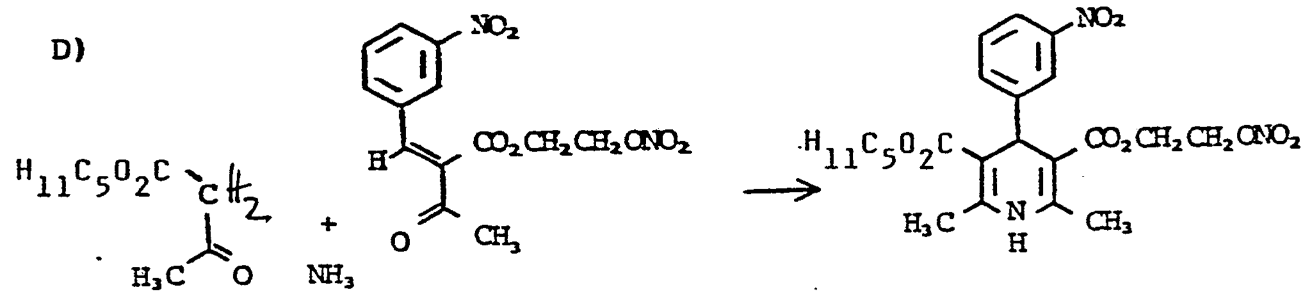

E)

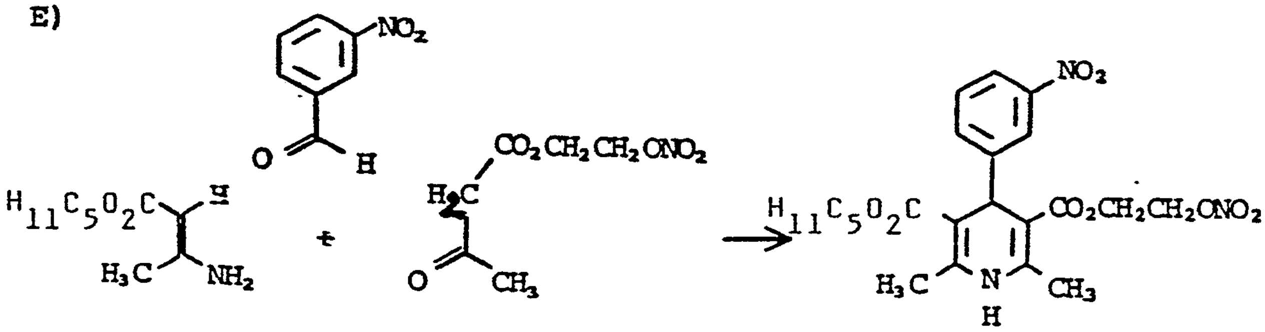

F)

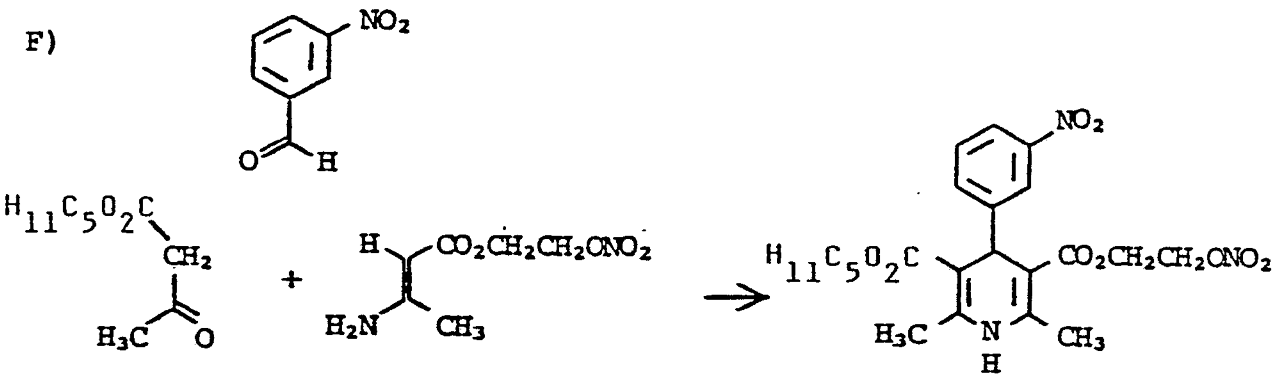

G)

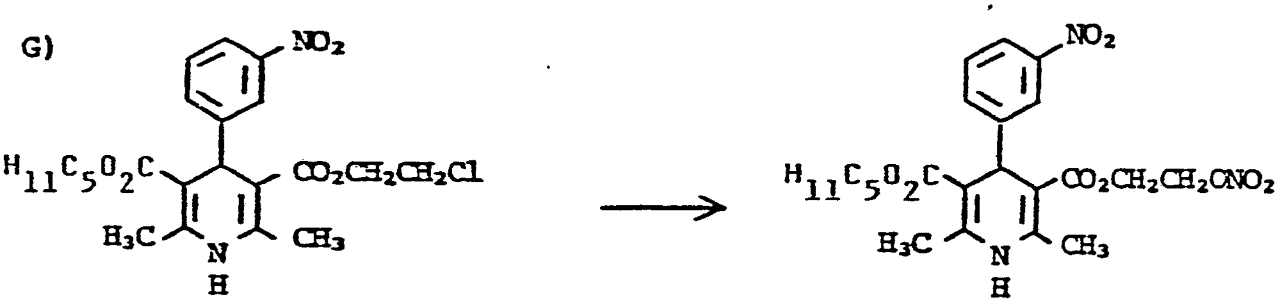

**Verfahrensvariante A und C**

Gemäß den Verfahrensvarianten A und C wird eine Ylidenverbindung der Formel II

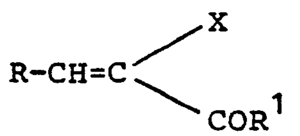

(II)

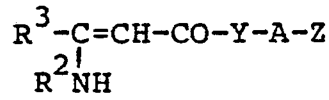

(III)

mit einem Enaminocarbonsäurederivat der Formel III oder eine Ylidenverbindung der Formel VI

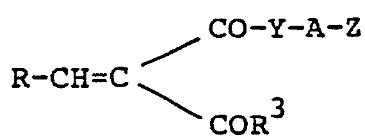

(VI)

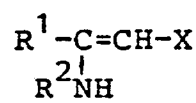

(VII)

mit einer Enaminoverbindung der Formel VII zur Reaktion gebracht.

Die als Ausgangsstoffe verwendeten Ylidenverbindungen der Formel II oder der Formel VI sind literaturbekannt oder können nach literaturbekannten Methoden hergestellt werden [vgl. z.B. G. Jones "The Knoevenagel Condensation" in Org. Reactions, Vol. XV , 204 f (1967)].

Als Beispiele seien genannt:

3,-Nitrobenzylidenacetessig-säuredecylester, 2'-Trifluormethylbenzylidenacetessig-säureisopropylester, 2'-Cyanobenzylidenacetessigsäurecyclopentylester, 2'-Chlorbenzylidenacetessigsäure-(2-methoxyethyl)-ester, 3'-Nitrobenzylidenacetessigsäure [2-(N-benzyl-N-methyl-amino)-ethyl]-ester, 3'-Nitrobenzylidenacetessigsäure-(2-nitrooxyethyl)-ester, 2',3'-Dichlorbenzylidenacetessigsäure-(2-nitrooxy-ethyl)-ester, α -Acetyl-β-(pyridyl-3)-acrylsäuremethylester, α -Acetyl-β-(pyridyl-3)-acrylsäure-(2-nitrooxyethyl)-ester, α-Acetyl-β-(benz-oxadiazolyl-4)-acrylsäure-(2-nitrooxyethyl)-ester, 3'-Nitrobenzylidenacetessigsäure-(2-nitrooxyethyl)-amid, 2',3-Dichlorbenzylidenacetessigsäure-(2-nitro-oxyethyl)-amid, α-Acetyl-β-(benzoxadiazolyl-4)-acetessigsäure-(2-nitrooxyethyl)-amid.

Die als Ausgangsstoffe verwendeten Enaminocarbonsäurederivate der Formel III und der Formel VII sind literaturbekannt oder können nach literaturbekannten Methoden hergestellt werden [vgl. A.G. Cope, J. Amer. Chem. Soc. 67, 1017 (1945)].

Als Beispiele seien genannt:

4-Amino-3-penten-2-on, β-Aminocrotonsäuremethylester, β-Aminocrotonsäuredecylester, β-Aminocrotonsäure-2,2,2-trifluor-ethylester, β-Aminocrotonsäure-(2-methoxyethyl)-ester, β-Aminocrotonsäurebenzylester, β-Aminocrotonsäure-(2-(Nbenzyl-N-methylamino)-ethyl)-ester, β-Aminocrotonsäure-(2-nitrooxyethyl)-ester, β-Aminocrotonsäure-(3-nitrooxypropyl)-ester.

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydroturan, Glykolmonomethylether, Glykoldimethylether oder Dimethylfomiamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise zwischen 20 und 100°C, insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol der Ylidenverbindung mit einem Mol Enaminocarbonsäurederivat in einem geeigneten Lösungsmittel zur Reaktion gebracht. Die Isolierung und Reinigung der erfindungsgemäßen Substanzen erfolgt vorzugsweise derart, daß man das Lösungsmittel im

Vakuum abdestilliert und den erhaltenen Rückstand aus einem geeigneten Lösungsmittel umkristallisiert oder einer der üblichen Reinigungsmethoden, wie beispielsweise der Säulenchromatographie an geeigneten Trägermaterialien, unterwirft.

**Verfahrensvariante B und D**

Gemäß den Verfahrensvariante B und D wird eine Ylidenverbindung der Formel II mit Aminen der Formel IV und β-Ketocarbonsäurederivaten der Formel V

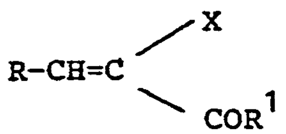

(II)

$R^2NH_2$

(IV)

$R^3-CO-CH_2-CO-Y-A-Z$

(V)

oder
eine Ylidenverbindung der Formel VI mit Aminen der Formel IV und β-Ketoderivaten der Formel VIII

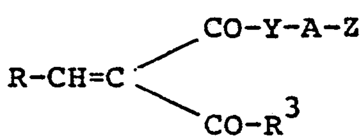

(VI)

$R^2-NH_2$

(IV)

$R^1CO-CH_2-X$

(VIII)

zur Reaktion gebracht.

In den Formeln II, IV, V, VI und VIII haben die Reste R, $R^1$, $R^2$, $R^3$, Y, A, Z und X die oben angegebene Bedeutung.

Beispiele für die als Ausgangssubstanzen verwendeten Ylidenverbindungen der Formel II und der Formel VI sind bereits unter den Verfahrensvarianten A und C aufgeführt worden.

Die erfindungsgemäß verwendbaren Amine der Formel IV sind bereits bekannt.

Als Beispiele seien genannt:

Amoniak, Methylanin, n-Butylamin, Isobutylamin oder Benzylamin.

Die als Ausgangsstoffe verwendeten β-Ketoderivate der Formel V und der Formel VIII sind bereits literaturbekannt oder können nach literaturbekannten Methoden hergestellt werden [z.B. D. Borrmann, "Umsetzung von Diketen mit Alkoholen, Phenolen und Mercaptanen", in Houben-Weyl, Methoden der Organischen Chemie, Vol. VII/4, 230 ff (1968); Y. Oikawa, K. Sugano und O. Yonemitsu, J. Org. Chem. 43, 2087 (1978)].

Als Beispiele seien genannt:

Acetylaceton, Acetessigsäuremethylester, Acetessigsäuredecylester, Acetessigsäurecyclopentylester, Acetessigsäure-2,2,2-trifluorethylester, Acetessigsäure-(2-methoxyethyl)-ester, Acetessigsäurebenzylester, Acetessigsäure-[2-(N-benzyl-N-methylamino)-ethyl)-ester, Acetessigsäure-(2-nitrooxyethyl)-ester, Acetessigsäure-(3-nitrooxypropyl)-ester, Acetessigsäure-(2-nitrooxyethyl)-amid.

Als Verdünmungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Dimethylformeid, Dinethylsulfoxid, Acetonitril, Pyridin und

Hexamethylphosphorsäuretriamid.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe jeweils in molaren Mengen eingesetzt.

**Verfahrensvarianten E und F**

Gemäß den Verfahrensvariante E und F wird ein Aldehyd der Formel IX mit einer Enaminoverbindung der Formel VII und einem β-Ketocarbonsäurederivat der Formel V

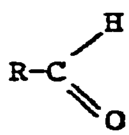

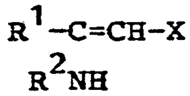

$R^3-CO-CH_2-CO-Y-A-Z$

(IX) (VII) (V)

oder

ein Aldehyd der Formel IX mit einem Enaminocarbonsäurederivat der Formel III und einem β-Ketoderivat der Formel VIII

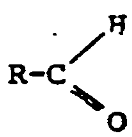

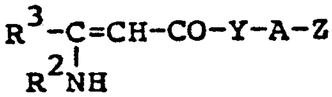

$R^1-CO-CH_2-X$

(IX) (III) (VIII)

zur Reaktion gebracht.

In den Formeln IX, VII, V, III und VIII haben die Reste R, $R^1$, $R^2$, $R^3$, Y, A, Z und X die oben angegebene Bedeutung.

Beispiele für die als Ausgangssubstanzen verwendeten Enaminoverbindungen der Formel VII und der Formel III sowie der β-Ketoderivate der Formel V und der Formel VIII sind bereits oben angeführt worden.

Die erfindungsgemäß verwendbaren Aldehyde der Formel IX sind literaturbekannt oder können nach literaturbekannten Methoden hergestellt werden [vgl. z.B. E. Mosettig, Org. Reactions VIII, 218 ff (1954)].

Als Beispiele seien genannt:

2-, 3- oder 4-Chlor/Brom/Fluorbenzaldehyd, 2-, 3- oder 4-Trifluormethylbenzaldehyd, 2-, 3- oder 4-Nitrobenzaldehyd, 2-, 3- oder 4-Cyanobenzaldehyd, 3-Azidobenzaldehyd, 2,3-, 2,4- oder 2,6-Dichlorbenzaldehyd, 2-Fluor-3-chlorbenzaldehyd, 2,4- oder 2,6-Dinitrobenzaldehyd, 2-Chlor-6-nitrobenzaldehyd, 4-Chlor-2-nitrobenzaldehyd, 2-Nitro-4-cyanobenzaldehyd, 2-Chlor-4-cyanobenzaldehyd, 3-Chlor-2-trifluormethylbenzaldehyd, Pyridin-2-, 3- oder 4-aldehyd, Benzoxadiazol-4-aldehyd.

Als Verdünnungsmittel kommen alle inerten organischen Lösungsmittel im Frage. Hierzu gehören vorzugsweise Alkohole wie Ethynol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril,

Pyridin und Hexamethylphosphorsäuretrianid.

Die Reaktionstemperaturen können in einem größeren Bereich variiert werden. Im allgemeinen arbeitet man zwischen 20 und 150° C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe jeweils in molaren Mengen eingesetzt.

**Verfahrensvariante G**

Bei der Verfahrensvariante G wird in 1,4-Dihydropyridin-derivaten der Formel X

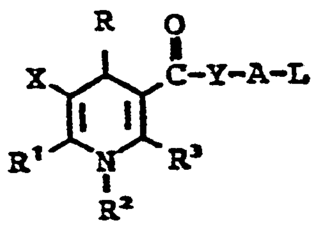

(X)

die austretende Gruppe L gegen den Rest $-ONO_2$ ausgetauscht.

In der Formel X haben die Reste R, $R^1$, $R^2$, $R^3$, X, Y und A die oben angegebene Bedeutung.

Die austretende Gruppe L steht in erster Linie für Halogen, insbesondere für Chlor, Brom oder Jod.

Die als Ausgangssubstanzen verwendeten 1,4-Dihydropyridin-derivate der Formel X sind literaturbekannt oder können nach literaturbekannten Methoden hergestellt werden (Deutsche Offenlegungsschrift 30 18 259).

Als Beispiele seien genannt:

1,4-Dihydro-2,6-dimethyl-4-(3-nitrophenyl)-pyridin-3,5-dicarbonsäure-(2-bromethyl)-cyclooentylester
1,4-Dihydro-2,6-dimethyl-4-(3-nitrophenyl)-pyridin-3,5-dicarbonsäure-(2-jodethyl)-decylester
1,4-Dihydro-2,6-dimethyl-4-(3-nitrophenyl)-pyridin-3,5-dicarbonsäure-(2-bromethyl)-benzylester
1,4-Dihydro-2,6-dimethyl-7-(3-hitrophenyl)-pyridin-3,5-dicarbonsäure-bis-(2-jodethyl)-ester
1,4-Dihydro-2,6-dimethyl-4-(2-trifluormethylphenyl)-pyridin-3,5-dicarbonsäure-(2-chlorethyl)-(2-methoxy-ethylester
1,4-Dihydro-2,6-dimethyl-4-(2,3-dichlorphenyl)-pyridin-3,5-dicarbonsäure-(2-bromethyl)-methylester
1,4-Dihydro-2,6-dimethyl-4-(2,1,3-benzoxadiazolyl-4)-pyridin-3,5-dicarbonsäure-(2-bromethyl)-isopropylester
1,4-Dihydro-2,6-dimethyl-4-(2,1,3-benzoxadiazolyl-4-)-pyridin-3,5-dicarbonsäure-(2-bromethyl)-ethylester.

Als Reaktionspartner dienen anorganische Nitrate, vorzugsweise Silbernitrat oder Quecksilber(I)- nitrat, wobei die Salze in bis zu fünffach molarem Überschuß eingesetzt werden können.

Die Reaktion kann sowohl heterogen als auch homogen durchgeführt werden. Als Verdünnungsmittel kommen alle hierfür inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150° C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Die vorstehenden Herstellungsverfahren sind lediglich zur Verdeutlichung angegeben, und die Herstellung der Verbindungen der allgemeinen Formel I ist nicht auf diese Verfahren beschränkt, sondern jede Modifikation dieser Verfahren ist in gleicher Weise für die Herstellung der erfindungsgemäßen Verbindungen anwendbar.

Je nach Wahl der Ausgaggssubstanzen können die erfindungsgemäßen Verbindungen in sterioisomeren Formen existieren, die sich entweder wie Bild und Spiegelbild (Enantiomere) oder die sich nicht wie Bild und Spiegelbild (Diastereomere) verhalten. Sowohl die Antipoden als auch die Racematformen als auch die Diastereomerengemische sind Gegenstand der Erfindung. Die Racemformen lassen sich ebenso wie die Diastereomeren in bekannter Weise in die stereoisomer einheitlichen Bestandteile trennen (vgl. z.B. E.L. Eliel, Stereochemistry of Carbon Compounds, McGraw Hill, 1962).

Außer den unten angeführten Herstellungsbeispielen seien folgende erfindungsgemäßen Wirkstoffe genannt:

1,4-Dihydro-2,6-dimethyl-4,(3-nitrophenyl)-pyridin-3,5-dicarbonsäure-(2-(N-benzyl-N-methylamino)-ethyl)-(2-nitrooxyethyl)-ester 1,4-Dihydro-2,6-dimethyl-4-(2,1,3-benzoxadiazolyl-4)-pyridin-3,5-dicarbonsäure-isopropyl-(2-nitrooxyethyl)-ester

Die neuen Verbindungen haben ein breites und vielseitiges pharmakologisches Wirkungsspektrum.

Im einzelnen konnten im Tierexperiment folgende Hauptwirkungen nachgewiesen werden:

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt.

Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäß wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. dem Zentralnervensystem) manifestieren. Die Verbindungen eignen sich daher besonders als Cerebraltherapeutika.

4. Die Verbindungen senken den Blutdruck von normotonen und hypertonen Tieren und können somit als antihypertensive Mittel verwendet werden.

5. Die Verbindungen haben stark muskulär-spasmolytische Wirkungen, die an der glatten Muskulatur des Magens, Darmtraktes, des Urogenitaltraktes und des Respirationssystems deutlich werden.

Die erfindungsgemäßen Verbindungen eignen sich aufgrund dieser Eigenschaften besonders zur Prophylaxe und Therapie der akuten und chronischen ischämischen Herzkrankheit im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder von Lösungsmitteln. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesam-Öl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emolgiermittel (z.B. Polyoxyethylen-Fettsäure-Ester, Polyoxyethylen-Fettalkohol-Ether, Alkylsulfonate und Arylsmlfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,05 bis 5 mg/kg Körpergewicht pro Tag zur Erzielumg wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

**Herstellungsbeispiele**

**Beispiel 1**

5-Acetyl-1,4-dihydro-2,6-dimethyl-4-(3-nitrophenyl)-pyridin-3-carbonsäure-(2-nitrooxyethyl)-ester

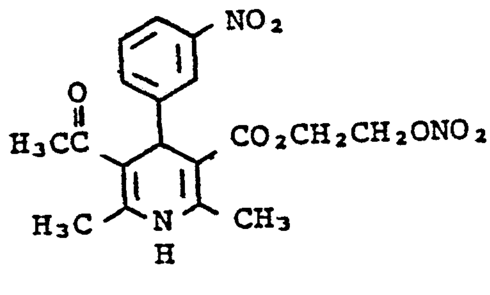

Eine Lösung von 14 g (36,9 mMol) 3-Acetyl-1,4-dihydro-2,6-dimethyl-4-(3-nitrophenyl)-pyridin-3-carbonsäure-(2-chlorethyl)-ester und 29,5 g (174 mMol) Silbernitrat in 220 ml Acetonitril wurde 2,5 Stunden unter Lichtausschluß bei 80°C gerührt. Das ausgefallene Silberchlorid wurde abgesaugt, das Filtrat im Vakuum eingeengt, der Rückstand in Dichlormethan aufgenommen umd kurz mit Eiswasser gewaschen. Nach Trocknen über wasserfreiem Natriumsulfat wurde die organische phase unter vermindertem Druck eingeengt. Der ölige Rückstand kristallisierte beim Verreiben mit wenig Ether durch, das Rohprodukt wurde abgesaugt und aus Methanol umkristallisiert.

Fp: 125-126°C, Ausbeute: 5,3 g (35 %)

**Beispiel 2**

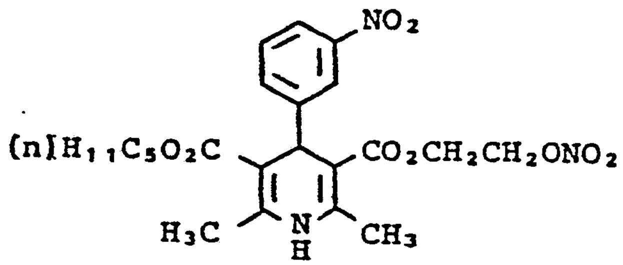

Analog Beispiel 1 wurde durch Umsetzung von 1,4-Dihydro-2,6-dimethyl-4-(3-nitrophenyl)-pyridin-3,5-dicarbonsäure-pentyl-(2-chlorethyl)-ester mit Silbernitrat in Acetonitril der 1,4-Dihydro-2,6-dimethyl-4-(3-nitrophenyl)-pyridin-3,5-dicarbonsäure-pentyl-(2-nitrooxyethyl)-ester vom Fp: 102°C erhalten. Ausbeute: 33 % der Theorie.

**Beispiel 3**

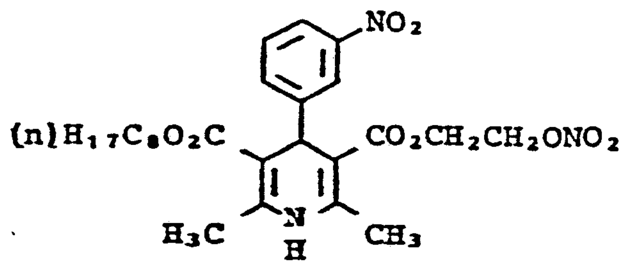

Analog Beispiel 1 wurde durch umsetzung von 1,4-Dihydro-2,6-dimethyl-4-(3-nitrophenyl)-pyridin-3,5-dicarbonsäure-octyl-(2-chlorethyl)-ester mit Silbernitrat in Acetonitril der 1,4-Dihydro-2,6-dinethyl-4-(3-nitrophenyl)-pyridin-3,5-dicarbonsäure-octyl-(2-nitrooxyethyl)-ester vom Fp: 96°C erhalten. Ausbeute: 25 % der Theorie.

**Beispiel 4**

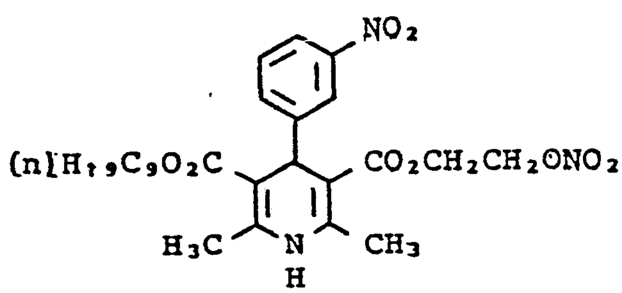

Analog Beispiel 1 wurde durch Umsetzung von 1,4-Dihydro-2,6-dimethyl-4-(3-nitrophenyl)-pyridin-3,5-dicarbonsäure-nonyl-(2-chlorethyl)-ester mit Silbernitrat in Acetonitril der 1,4-Dihydro-2,6-dimethyl-4-(3-nitrophenyl)-pyridin-3,5-dicarbonsäure-nonyl-(2-nitrooxyethyl)-ester vom Fp: 89°C erhalten. Ausbeute: 31 % der Theorie.

**Beispiel 5**

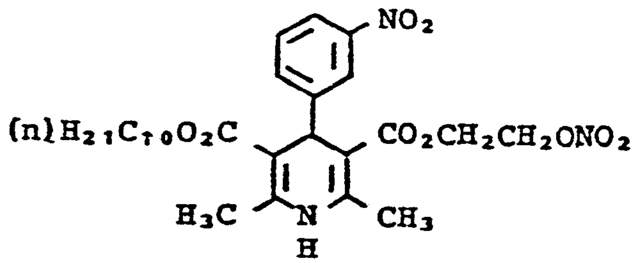

Analog Beispiel 1 wurde durch Umsetzung von 1,4-Dihydro-2,6-dimethyl-4-(3-nitrophenyl)-pyridin-3,5-dicarbonsäure-decyl-(2-chlorethyl)-ester mit Silbernitrat in Acetonitril der 1,4-Dihydro-2,6-dimethyl-4-(3-nitrophenyl)-pyridin-3,5-dicarbonsäure-decyl-(2-nitrooxyethyl)-ester vom Fp: 96°C erhalten. Ausbeute: 22 % der Theorie.

**Beispiel 6**

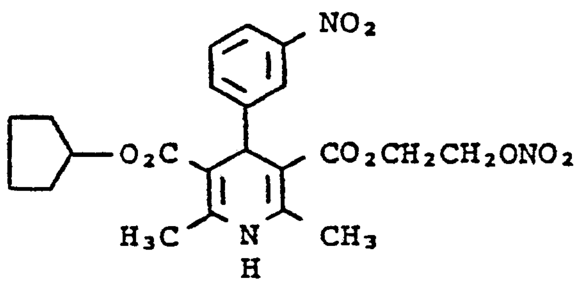

Analog Beispiel 1 wurde durch Umsetzung von 1,4-Dihydro-2,6-dimethyl-4-(3-nitrophenyl)-pyridin-3,5-dicarbonsäure-cyclopentyl-(2-chlorethyl)-ester mit Silbernitrat in cetonitril der 1,4-Dihydro-2,6-dimethyl-4-(3-nitrophenyl)-yridin-3,5-dicarbonsäure-cyclopentyl-(2-nitrooxyethyl)-ster vom Fp: 140°C erhalten. Ausbeute: 39 % der Theorie.

**Beispiel 7**

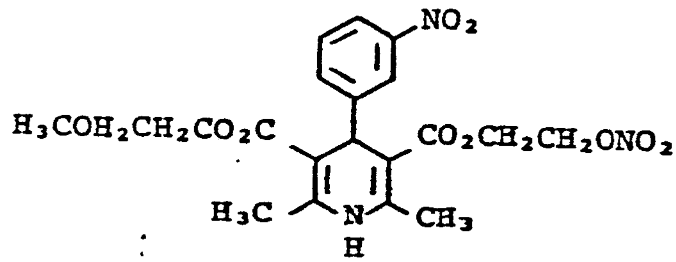

Analog Beispiel 1 wurde durch Umsetzung von 1,4-Dihydro-2,6-dimethyl-4-(3-nitrophenyl)-pyridin-3,5-dicarbonsäure-(2-methoxy ethyl)-(2-chlorethyl)-ester mit Silbernitrat Acetonitril der 1,4-Dihydro-2,6-dimethyltl-4-(3-nitro-phenyl)-pyridin-3,5-dicarbonsäure-t2-methoxyethyl)-(2-nitrooxyethyl)-ester vom FP: 118°C erhalten. Ausbeute: 48 % der Theorie.

**Beispiel 8**

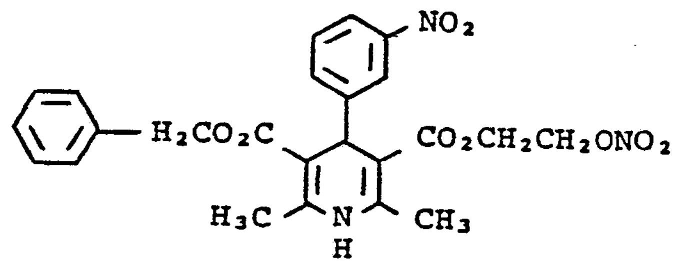

Analog Beispiel 1 wurde durch Umsetzung vom 1,4-Dihydro-2,6-dimethyl-4-(3-nitrophenyl)-pyridin-3,5-dicarbonsäure-benzyl-(2-chlorethyl)-ester mit Silbernitrat in Acetonitril der 1,4-Dihydro-2,6-dimethyl-4-(3-nitrophenyl)-pyridin-3,5-dicarbonsäure-benzyl-(2-nitrooxyethyl)-ester vom Fp: 137°C erhalten. Ausbeute: 34 % der Theorie.

**Beispiel 9**

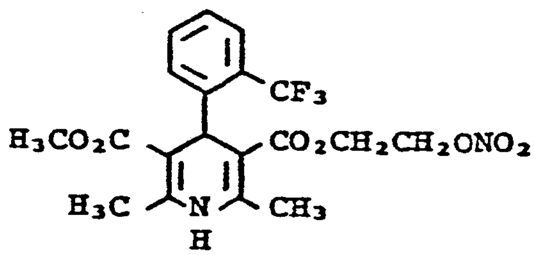

Analog Beispiel 1 wurde durch Umsetzung von 1,4-Dihydro-2,6-dimethyl-4-(2-trifluormethylphenyl)-pyridin-3,5-di-carbonsäure-methyl-(2-chlorethyl)-ester mit Silbernitrat in Acetonitril der 1,4-Dihydro-2,6-dimethyl-4-(2-trifluormethylphenyl)-pyridin-3,5-dicarbonsäure-methyl-(2-nitrooxyethyl)-ester vom Fp: 123°C erhalten. Ausbeute: 34 % der Theorie.

**Beispiel 10**

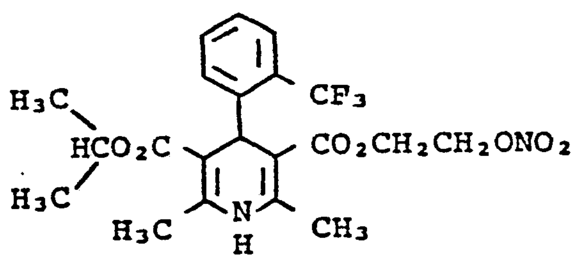

Analog Beispiel 1 wurde durch Umsetzung von 1,4-Dihydro-2,6-dimethyl-4-(2-trifluormethylphenyl)-pyridin-3,5-di-carbonsäure-isopropyl-(2-chlorethyl)-ester mit Silbernitrat in Acetonitril der 1,4-Dihydro-2,6-dimethyl-4-(2-trifluormethylphenyl)-pyridin-3,5-dicarbonsäure-isopro-pyl-(2-nitrooxyethyl)-ester vom Fp: 114°C erhalten. Ausbeute: 29 % der Theorie.

**Patentansprüche**

1. 1,4-Dihydropyridine der allgemeinen Formel I

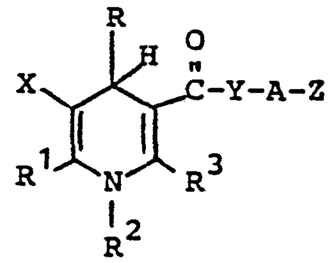

(I)

in welcher
R für Pyridyl, Benzoxadiazolyl oder Phenyl steht, welches 1- bis 2-fach substituiert ist durch Nitro, Trifluormethyl, Halogen, Cyano und/oder Azido,
$R^1$ und $R^3$ gleich oder verschieden sind und jeweils $C_1$-$C_4$-Alkyl oder Benzyl bedeuten,
$R^2$ Wasserstoff, $C_1$-$C_4$-Alkyl oder Benzyl darstellt,
Y Sauerstoff ist,
A $C_1$-$C_4$-Alkylen bedeutet,
Z $-ONO_2$ darstellt,
X für $-COR^4$ mit $R^4$ gleich $C_1$-$C_4$-Alkyl, oder für $-COOR^5$ steht, wobei $R^5$ Benzyl oder geradkettiges, verzweigtes oder cyclisches Alkyl mit bis zu 12 C-Atomen bedeutet, das gegebenenfalls durch ein Sauerstoffatom in der Alkylkette unterbrochen ist oder gegebenenfalls substituiert ist durch Halogen und/oder durch Amino, wobei die Aminogruppe zwei gleiche oder verschiedene Substituenten aus der Gruppe $C_1$-$C_4$-Alkyl und Benzyl aufweist, wobei für den Fall, daß R für Nitrophenyl steht, $R^5$ mindestens 5 C-Atome enthält, oder
für die Gruppe -CO-Y'-A'-Z' steht, wobei diese Gruppe mit -CO-Y-A-Z gleich oder verschieden sein kann und wobei die Definition von Y', A' und Z' der von Y, A und Z entspricht,
sowie deren pharmazeutisch unbedenklichen Säureadditionssalze.

2. 1,4-Dihydropyridine gemäß Anspruch 1, in welchen
R Nitrophenyl oder Trifluormethylphenyl bedeutet,
$R^1$ und $R^3$ Methyl darstellen,
$R^2$ Wasserstoff ist,
Y Sauerstoff bedeutet,
A $C_1$-$C_3$-Alkylen darstellt,
Z $-ONO_2$ ist,
X $-COR^4$ mit $R^4$ gleich Methyl bedeutet oder für $-COOR^5$ steht, wobei $R^5$ geradkettiges, verzweigtes oder cyclisches Alkyl mit bis zu 12 C-Atomen, gegebenenfalls durch ein Sauerstoffatom in der Alkylkette unterbrochen, oder Benzyl darstellt, wobei für den Fall, daß R für Nitrophenyl steht, $R^5$ mindestens 5 C-Atome enthält.

3. Verfahren zur Herstellung von 1,4-Dihydropyridinen der allgemeinen Formel I

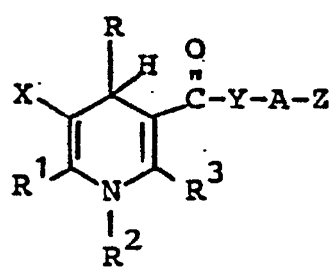

(I)

in welcher
R für Pyridyl, Benzoxadiazolyl oder Phenyl steht, welches 1- bis 2-fach substituiert ist durch Nitro, Trifluormethyl, Halogen, Cyano und/oder Azido,
$R^1$ und $R^3$ gleich oder verschieden sind und jeweils $C_1$-$C_4$-Alkyl oder Benzyl bedeuten,

$R^2$ Wasserstoff, $C_1$-$C_4$-Alkyl oder Benzyl darstellt,
Y Sauerstoff ist,
A $C_1$-$C_4$-Alkylen bedeutet,
Z $-ONO_2$ darstellt,
X für $-COR^4$ mit $R^4$ gleich $C_1$-$C_4$-Alkyl, oder für $-COOR^5$ steht, wobei $R^5$ Benzyl oder geradkettiges, verzweigtes oder cyclisches Alkyl mit bis zu 12 C-Atomen bedeutet, das gegebenenfalls durch ein Sauerstoffatom in der Alkylkette unterbrochen ist oder gegebenenfalls substituiert ist durch Halogen und/oder durch Amino, wobei die Aminogruppe zwei gleiche oder verschiedene Substituenten aus der Gruppe $C_1$-$C_4$-Alkyl und Benzyl aufweist, wobei für den Fall, daß R für Nitrophenyl steht $R^5$ mindestens 5 C-Atome enthält, oder
für die Gruppe -CO-Y'-A'-Z' ateht, wobei diese Gruppe mit -CO-Y-A-Z gleich oder verschieden sein kann und wobei die Definition von Y', A' und Z' der von Y, A und Z entspricht,
dadurch gekennzeichnet, daß man
A) Ylidenverbindungen der Formel II

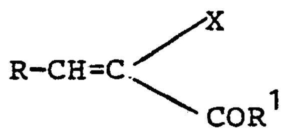
(II)

in welcher
R, $R^1$ und X die oben angegebene Bedeutung haben,
mit Enaminocarbonsäurederivaten der Formel III,

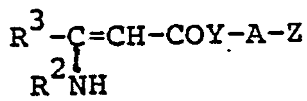
(III)

in welcher
$R^2$, $R^3$, Y, A und Z die oben angegebene Bedeutung haben,
gegebenenfalls in Gegenwart inerter organischer Lösungsmittel umsetzt, oder
B) Ylidenverbindungen der Formel II

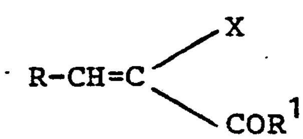
(II)

in welcher
R, $R^1$ und X die oben angegebene Bedeutung haben,
mit Aminen der Formel IV und β-Ketocarbonsäurederivaten der Formel V

$R^2-NH_2$ $R^3-CO-CH_2-COY-A-Z$

(IV) (V)

in welchen
$R^2$, $R^3$, Y, A und Z die oben angegebene Bedeutung haben,
gegebenfalls in Gegenwart inerter organischer Lösungsmittel umsetzt, oder
C) Yliden -β-dicarbonylverbindungen der Formel VI

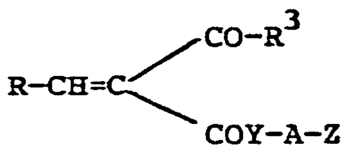

(VI)

in welcher
R, $R^3$, Y, A und Z die oben angegebene Bedeutung haben,
mit Enaminoverbindungen der Formel VII

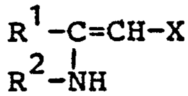

(VII)

in welcher
$R^1$, $R^2$ und X die oben angegebene Bedeutung haben,
gegebenenfalls in Gegenwart inerter organischer Lösungsmittel zur Reaktion bringt, oder
D) Yliden-β-dicarbonylverbindungen der Formel VI

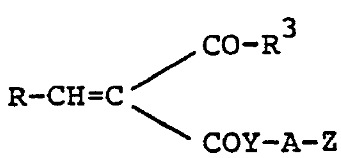

(VI)

in welcher
R, $R^3$, Y, A und Z die oben angegebene Bedeutung haben,

mit Aminen der Formel IV und Keto -Derivaten der Formel VIII

$R^2-NH_2$ (IV)

$R^1-CO-CH_2-X$ (VIII)

in welchen
$R^2$, $R^1$ und X die oben angegebene Bedeutung haben,
gegebenenfalls in Gegenwart inerter organischer Lösungsmittel umsetzt, oder
E) Aldehyde der Formel IX,

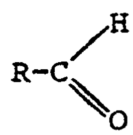

(IX)

in welcher
R die oben angegebene Bedeutung hat,
mit Enaminoverbindungen der Formel VII

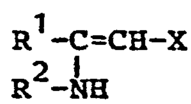

(VII)

in welcher
$R^1$, $R^2$ und X die oben angegebene Bedeutung haben,
und β-Ketocarbonsäurederivaten der Formel V

$R^3-CO-CH_2-COY-A-Z$ (V)

in welcher
$R^3$, Y, A und Z die oben angegebene Bedeutung haben,
gegebenenfalls in Gegenwart inerter organischer Lösungsmittel umsetzt, oder

F) Aldehyde der Formel IX

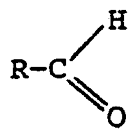

(IX)

in welcher R die oben angegebene Bedeutung hat, mit Enaminocarbonsäurederivaten der Formel III

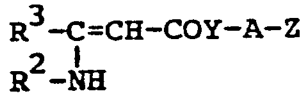

(III)

in welcher $R^2$, $R^3$, Y, A und Z die oben angegebene Bedeutung haben, und Keto-Derivaten der Formel VIII

$$R^1\text{-CO-CH}_2\text{-X} \qquad \text{(VIII)}$$

in welcher $R^1$ und X die oben angegebene Bedeutung haben, gegebenenfalls in Gegenwart inerter organischer Lösungsmittel umsetzt, oder daß man

G) in 1,4-Dihydropyridinderivaten der Formel X

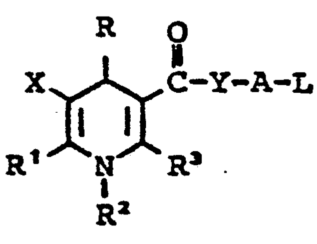

(X)

in welcher R, $R^1$, $R^2$, $R^3$, A, X und Y die oben angegebene Bedeutung haben und L eine geeignete austretende Gruppe, wie Halogen darstellt, diese Gruppe L gegen den Rest -O-$NO_2$ austauscht.

4. 1,4-Dihydropyridine gemäß Ansprüchen 1 und 2 zur Verwendung bei der Bekämpfung von Erkrankungen.

5. 1,4-Dihydropyridine gemäß Ansprüchen 1 und 2 zur Verwendung bei der Bekämpfung von Kreislauferkrankungen.

6. Arzneimittel enthaltend als Wirkstoff eine oder mehrere Verbindungen gemäß Ansprüchen 1 und 2.

7. Verfahren zur Herstellung von Arzneimitteln enthaltend eine oder mehrere Verbindungen der allgemeinen Formel gemäß Anspruch 1, dadurch gekennzeichnet, daß man die entsprechenden 1,4-Dihydropyridine mit inerten nicht-toxischen pharmazeutisch geeigneten Trägerstoffen oder Lüsungsmitteln vermischt.

8. Verwendung von 1,4-Dihydropyridinen der allgemeinen Formel gemäß Anspruch 1, zur Herstellung von Kreislaufmitteln.

## Claims

1. 1,4-Dihydropyridines of the general formula I

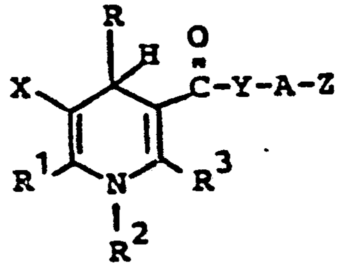

(I)

in which

R represents pyridyl, benzoxadiazolyl or phenyl, which is mono to disubstituted by nitro, trifluoromethyl, halogen, cyano and/or azido,

$R^1$ and $R^3$ are identical or different and each denote $C_1$-$C_4$-alkyl or benzyl,

$R^2$ represents hydrogen, $C_1$-$C_4$-alkyl or benzyl,

Y is oxygen,

A denotes $C_1$-$C_4$-alkylene,

Z represents $-ONO_2$

X represents $-COR^4$, in which $R^4$ is $C_1$-$C_4$-alkyl, or $-COOR^5$, wherein $R^5$ denotes benzyl or straight-chain, branched or cyclic alkyl with up to 12 C atoms, which is optionally interrupted by an oxygen atom in the alkyl chain or is optionally substituted by halogen and/or by amino, the amino group containing two identical or different substituents from the group comprising $C_1$-$C_4$-alkyl and benzyl, wherein in the case where R reoresents nitrophenyl $R^5$ contains at least 5 C atoms, or

represents the group -CO-Y'-A'-Z', it being possible for this group to be identical to or different from -CO-Y-A-Z and the definition of Y', A' and Z' corresponding to that of Y, A and Z,

and their pharmaceutically acceptable acid addition salts.

2. 1,4-Dihydropyridines according to Claim 1, in which

R denotes nitrophenyl or trifluoromethylphenyl,

$R^1$ and $R^3$ each represent methyl,

$R^2$ is hydrogen,

Y denotes oxygen,

A represents $C_1$-$C_3$-alkylene,

Z is $-ONO_{2i}$

X denotes $-COR^4$, in which $R^4$ denotes methyl, or represents $-COOR^5$, wherein $R^5$ represents straightchain, branched or cyclic alkyl with up to 12 C atoms, opLionally interrupted by an oxygen atom in the-alkyl chain, or benzyl, wherein in the case where R represents nitrophenyl, $R^5$ contains at least 5 C atoms.

3. Process for the preparation of 1,4-dihydropyridines of the general formula I

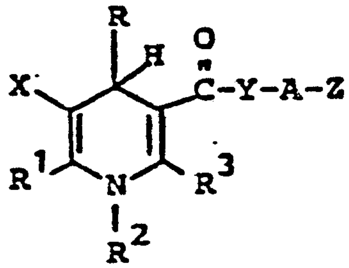 (I)

in which
R represents pyridyl, benzoxadiazolyl or phenyl, which is mono- to disubstituted by nitro, trifluoromethyl, halogen, cyano and/or azido,
$R^1$ and $R^3$ are identical or different and each denote $C_1$-$C_4$-alkyl or benzyl,
$R^2$ represents hydrogen, $C_1$-$C_4$-alkyl or benzyl,
Y is oxygen,
A denotes $C_1$-$C_4$-alkylene,
Z represents $-ONO_2$,
X represents $-COR^4$, in which $R^4$ is $C_1$-$C_4$-alkyl, or $-COOR^5$, wherein $R^5$ denotes benzyl or straight-chain, branched-or cyclic alkyl with up to 12 C atoms, which is optionally interrupted by an oxygen atom in the alkyl chain or is optionally substituted by halogen and/or by amino, the amino group containing two identical or different substituents from the group comprising $C_1$-$C_4$-alkyl and benzyl, wherein in the case where R represents nitrophenyl $R^5$ contains at least 5 C atoms, or
represents the group -CO-Y'-A'-Z',-it being possible for this group to be identical to or different from -CO-Y-X-Z and the definition of Y', A' and Z' corresponding to that of Y, A and Z,
characterized in that A) ylidene compounds-of the formula II

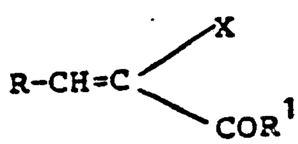 (II)

in which
R, $R^1$ and X have the abovementioned meaning, are reacted with enaminocarboxylic acid derivatives of the formula III

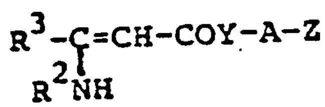 (III)

in which
$R^2$, $R^3$, Y, A and Z have the abovementioned meaning, if appropriate in the presence of inert organic solvents, or

B) ylidene compounds of the formula II

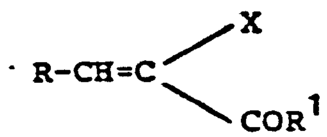

(II)

in which
R, $R^1$ and X have the abovementioned meaning, are reacted with amines of the formula IV and β-ketocarboxylic acid derivatives of the formula V

$R^2-NH_2$ (IV)

$R^3-CO-CH_2-COY-A-Z$ (V)

in which
$R^2$, $R^3$, Y, A and Z have the abovementioned meaning, if appropriate in the presence of inert organic solvents, or
C) ylidene-β-dicarbonyl compounds of the formula VI

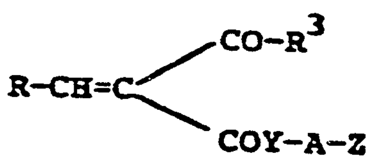

(VI)

in which
R, $R^3$, Y, A and Z have the abovementioned meaning, are reacted with enamino compounds of the Formula VII

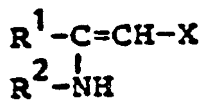

(VII)

in which
$R^1$, $R^2$ and X have the abovementioned meaning, if appropriate in the presence of inert organic solvents, or
D) ylidene-β-dicarbonyl compounds of the formula VI

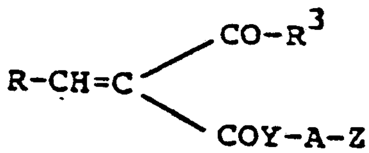

(VI)

in which
R, $R^3$, Y, A and Z have the abovementioned meaning, are reacted with amines of the formula IV and keto derivates of the formula VIII

$$R^2-NH_2$$

(IV)

$$R^1-CO-CH_2-X$$

(VIII)

in which
$R^2$, $R^1$ and X have the abovementioned meaning, if appropriate in the presence of inert organic solvents, or
E) aldehydes of the Formula IX

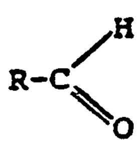

(IX)

in which
R has the abovementioned meaning, are reacted with enamino compounds of the Formula VII

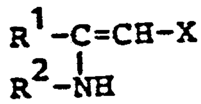

(VII)

in which
$R^1$, $R^2$ and X have the abovementioned meaning, and β-ketocarboxylic acid derivatives of the Formula V

$$R^3-CO-CH_2-COY-A-Z \qquad (V)$$

in which
$R^3$, Y, A and Z have the abovementioned meaning, if appropriate in the presence of inert organic solvents, or
F) aldehydes of the formula IX

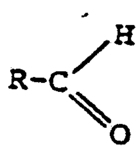

(IX)

in which
R has the abovementioned meaning, are reacted with enaminocarboxylic acid derivates of the formula III

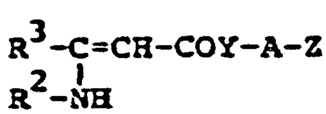

(III)

in which
$R^2$, $R^3$, Y, A and Z have the abovementioned meaning, and keto derivatives of the formula VIII

$$R^1-CO-CH_2-X \qquad (VIII)$$

in which
$R^1$ and X have the abovementioned meaning, if appropriate in the presence of inert organic solvents, or in

that

G) in 1,4-dihydropyridine derivatives of the formula X

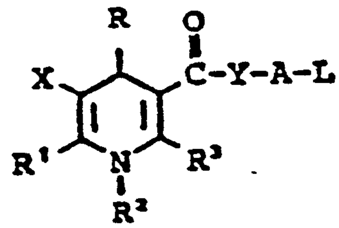

(X)

in which

R, $R^1$, $R^2$, $R^3$, A, X and Y have the abovementioned meaning and

L represents a suitable leaving group,

this group L is replaced by the radical $-O-NO_2$.

4. 1,4-Dihydropyridines according to Claims 1 and 2 for use in combating diseases.

5. 1,4-Dihydropyridines according to Claims 1 and 2 for use in combating circulatory diseases.

6. Medicaments containing, as the active compound, one or more compounds according to Claims 1 and 2.

7. Process for the preparation of medicaments containing one or more compounds of the general formula I according to Claim 1, characterised in that the corresponding 1,4-dihydro-pyridines are mixed with inert nontoxic pharmaceutically suitable excipients or solvents.

8. Use of 1,4-dihydropyridines of the general formula I according to Claim 1 for the preparation of cardiovascular agents.

**Revendications**

1. 1,4-dihydropyridines de formule générale

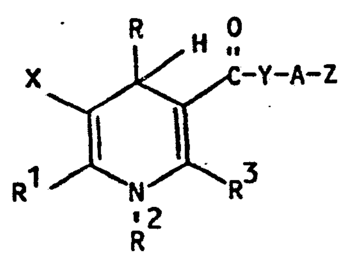

(I)

dans laquelle

R représente un reste pyridyle, benzoxadiazolyle ou phényle mono- à di-substitué par des groupes nitro, trifluorométhyle, des halogènes, des groupes cyano et/ou azido,

$R^1$ et $R^3$, ayant des significations identiques ou différentes, représentent chacun un groupe alkyle en $C_1$-$C_4$ ou benzyle,

$R^2$ représente l'hydrogène, un groupe alkyle en $C_1$-$C_4$ ou benzyle,

Y représente l'oxygène,

A représente un groupe alkylène en $C_1$-$C_4$,

Z représente $-ONO_2$,

X représente $-COR^4$ dans lequel $R^4$ est un groupe alkyle en $C_1$-$C_4$, ou $-COOR^5$ dans lequel $R^5$ représente un groupe benzyle ou un groupe alkyle à chaîne droite, ramifiée ou cyclique contenant jusqu'à 12 atomes de carbone, qui peut le cas échéant être interrompu par un atome d'oxygène dans la chaîne alkyle ou qui est le cas échéant substitué par des halogènes et/ou un groupe amino, le groupe amino portant deux substituants

identiques ou différents choisis parmi les groupes alkyle en $C_1$-$C_4$ et benzyle, étant spécifié que, dans le cas où R représente un groupe nitrophényle, $R^5$ contient au moins 5 atomes de carbone, ou bien

le groupe -CO-Y'-A'-Z', ce groupe pouvant être identique à -CO-Y-A-Z ou différent de celui-ci, les définitions de Y', A' et Z' correspondant à celles de Y, A et Z,

et leurs sels formés par addition avec des acides acceptables pour l'usage pharmaceutique.

2. 1,4-dihydropyridines selon la revendication 1, dans lesquelles
R représente un groupe nitrophényle ou trifluorométhylphényle,
$R^1$ et $R^3$ représentent chacun un groupe méthyle,
$R^2$ représente l'hydrogène,
Y représente l'oxygène,
A représente un groupe alkylène en $C_1$-$C_3$,
Z représente $-ONO_2$,
X représente $-COR^4$ dans lequel $R^4$ est un groupe méthyle ou $-COOR^5$ dans lequel $R^5$ représente un groupe alkyle à chaîne droite, ramifiée ou cyclique contenant jusqu'à 12 atomes de carbone, éventuellement interrompu par un atome d'oxygène dans la chaîne alkyle, ou un groupe benzyle, étant spécifié que, dans le cas où R représente un groupe nitrophényle, $R^5$ contient au moins 5 atomes de carbone.

3. Procédé de préparation des 1,4-dihydropyridines de formule générale I

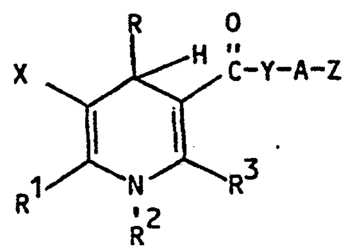

(I)

dans laquelle
R représente un groupe pyridyle, benzoxadiazolyle ou phényle mono- à di-substitué par des groupes nitro, trifluorométhyle, des halogènes, des groupes cyano et/ou azido,
$R^1$ et $R^3$, ayant des significations identiques ou différentes, représentent chacun un groupe alkyle en $C_1$-$C_4$ ou benzyle,
$R^2$ représente l'hydrogène, un groupe alkyle en $C_1$-$C_4$ ou benzyle,
Y représente l'oxygène,
A represente un groupe alkylène en $C_1$-$C_4$,
Z représente $-ONO_2$,
X représente $-COR^4$ dans lequel $R^4$ représente un groupe alkyle en $C_1$-$C_4$, ou $-COOR^5$ dans lequel $R^5$ représente un groupe benzyle ou un groupe alkyle à chaîne droite, ramifiée ou cyclique contenant jusqu'à 12 atomes de carbone, qui peut le cas échéant être interrompu par un atome d'oxygène dans la chaîne alkyle, ou qui est éventuellement substitué par des halogènes et/ou un groupe amino, le groupe amino portant deux substituants identiques ou différents choisis parmi les groupes alkyle en $C_1$-$C_4$ et benzyle, étant spécifié que, lorsque $R^2$ represente un groupe nitrophényle, $R^5$ contient au moins 5 atomes de carbone, ou bien le groupe -CO-Y'-A'-Z', ce groupe pouvant être identique à -CO-Y-A-Z ou différent de celui-ci, les definitions de Y', A' et Z' correspondant à celles de Y, A et Z, caractérisé en ce que

A) on fait réagir des composés en ylidène de formule II

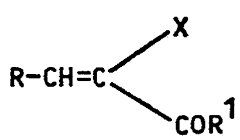

(II)

dans laquelle R, $R^1$ et X ont les significations indiquées ci-dessus, avec des dérivés d'acides énaminocarboxyliques de formule III

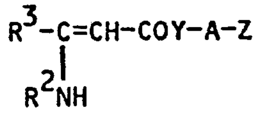

(III)

dans laquelle $R^2$, $R^3$, Y, A et Z ont les significations indiquées ci-dessus, éventuellement en présence de solvants organiques inertes, ou bien

B) on fait réagir des composés en ylidène de formule II

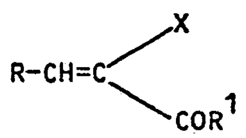

(II)

dans laquelle R, $R^1$ et X ont les significations indiquées ci-dessus, avec des amines de formule IV et des dérivés d'acides β-cétocarboxyliques de formule V

$R^2-NH_2$
(IV)

$R^3-CO-CH_2-COY-A-Z$
(V)

dans lesquelles $R^2$, $R^3$, Y, A et Z ont les significations indiquées ci-dessus, éventuellement en présence de solvants organiques inertes, ou bien

C) on fait réagir des composés ylidène-β-dicarbonylés de formule VI

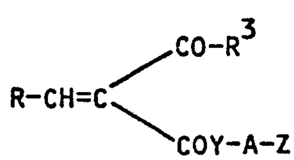

(VI)

dans laquelle R, $R^3$, Y, A et Z ont les significations indiquées ci-dessus, avec des composés énaminés de formule VII

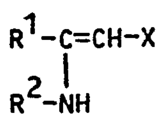

(VII)

dans laquelle $R^1$, $R^2$ et X ont les significations indiquées ci-dessus, éventuellement en présence de solvants organiques inertes, ou bien

D) on fait réagir des composés ylidène-β-dicarbonylés de formule VI

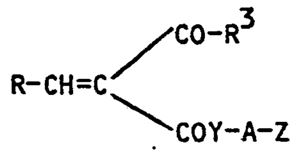

(VI)

dans laquelle R, $R^3$, Y, A et Z ont les significations indiquées ci-dessus, avec des amines de formule IV et des dérivés cétoniques de formule VIII

$R^2-NH_2$
(IV)

$R^1-CO-CH_2-X$
(VIII)

dans lesquelles $R^2$, $R^1$ et X ont les significations indiquées ci-dessus, éventuellement en présence de solvants organiques inertes, ou bien

E) on fait réagir des aldéhydes de formule IX

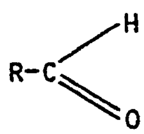

(IX)

dans laquelle R a les significations indiquées ci-dessus, avec des composés énaminés de formule VII

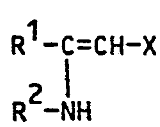

(VII)

dans laquelle $R^1$, $R^2$ et X ont les significations indiquées ci-dessus, et des dérivés d'acides β-cétocarboxyliques de formule V

$R^3-CO-CH_2-COY-A-Z$ (V)

dans laquelle $R^3$, Y, A et Z ont les significations indiquées cidessus, éventuellement en présence de solvants organiques inertes, ou bien

F) on fait réagir des aldéhydes de formule IX

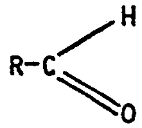

(IX)

dans laquelle R a les significations indiquées ci-dessus, avec des dérivés d'acides énaminocarboxyliques de formule III

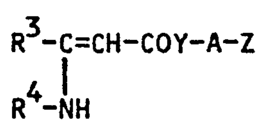

(III)

dans laquelle $R^2$, $R^3$, Y, A et Z ont les significations indiquées ci-dessus, et des dérivés cétoniques de formule VIII

$$R^1-CO-CH_2-X \qquad (VIII)$$

dans laquelle $R^1$ et X ont les significations indiquées ci-dessus, éventuellement en présence de solvants organiques inertes, ou bien

G) on part de dérivés de la 1,4-dihydropyridine répondant à la formule X

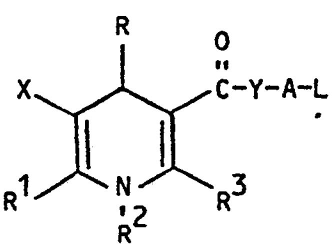

(X)

dans laquelle R, $R^1$, $R^2$, $R^3$, A, X et Y ont les significations indiquées ci-dessus et L représente un groupe éliminable approprié, et on échange ce groupe L contre le reste $-O-NO_2$.

4. 1,4-dihydropyridines selon les revendications 1 et 2, pour l'utilisation dans le traitement de maladies.

5. 1,4-dihydropyridines selon les revendications 1 et 2, pour l'utilisation dans le traitement de maladies de la circulation.

6. Médicament contenant en tant que substance active un ou plusieurs composés selon les revendications 1 et 2.

7. Procédé de préparation de médicaments contenant un ou plusieurs composés de formule générale I, selon la revendication 1, caractérisé en ce que l'on mélange les 1,4-dihydropyridines en question avec des véhicules ou solvants inertes, non toxiques, convenant pour l'usage pharmaceutique.

8. Utilisation des 1,4-dihydropyridines de formule générale 1 selon la revendication 1, pour la préparation de médicaments agissant sur la circulation.